# EUROPEAN PATENT APPLICATION

(11) **EP 1 972 324 A1**
(43) Date of publication of application: **24.09.2008**
(21) Application number: 07005748.4
(22) Date of filing: 21.03.2007
(51) Int. Cl.: A61K 8/11, A61K 8/55, A61Q 5/00, A61Q 5/02, A61Q 19/00, A61Q 19/10

(54) **Encapsulated liposomes**

(71) Applicant: Cognis IP Management GmbH, 40589 Düsseldorf (DE)
(72) Inventor: Viladot Petit, Josep-Lluis, 08018 Barcelona (ES); Asensio, Juan-Antonio, 08820 El Prat de Llobregat (ES); Gomez, Yolanda, 08170 Montornés del Vallés (ES)
(74) Representative: Fabry, Bernd

(57) **Abstract**

Encapsulated liposomes and method for production thereof whereby:
(a) actives are brought into contact with agents capable to form liposomes, and
(b) the liposomes thus obtained are treated with polymers or surfactants showing the opposite charge of said liposomes to form capsules,
for use in cosmetic, pharmaceutical, personal care, detergent or food compositions, as well as textile or paper additives and lacquers and paints.

## Description

### Field of the invention

The present invention is related to the area of micro- or nanocapsules and refers to encapsulated liposomes, useful for various areas of application.

### Background of the invention

Nancapsules or microcapsules are understood to be spherical aggregates with a diameter of about a few nanometers to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone. The active principles are released from the microcapsules by mechanical, thermal, chemical or enzymatic destruction of the membrane, normally during the use of the preparations containing the microcapsules.

Examples of known microcapsules are the following commercial products (the membrane material is shown in brackets) Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids). Therefore it is fact that the state of the art discloses numerous types of encapsulation systems which are useful for very different purposes. Nevertheless, there is still a strong need for capsules serving very special needs. For example, there are microcapsules in the market which show a suitable stability and flexibility; however the average diameter is too broad for the application in certain cosmetic or pharmaceutical areas. On the other hand rather small particles are obtainable which however do not exhibit the stability over a longer storage time. Others show the right particle size but one finds it difficult to break the shells and to release the active.

Therefore the object of the present invention has been to develop a special type of micro- or nanocapsule which fulfils the following requirements:
o An average particle size of 10 to 900 nm;
o A small particle size distribution, where preferably at least 70 % of the particles shown a diameter of 100 to 1.200 nm;
o An inner phase comprising the active and a lipophilic carrier in order to facilitate the transport of the active into the stratum corneum or the keratin fibre;
o A flexible shell which shows a sufficient hardness for storing the capsules even in the presence of surfactants, but breaks easily under mechanic pressure.
o Preferably a positive charged surface which makes it easy to bind the capsules to fibres.

### Detailed description of the invention

The present invention refers to encapsulated liposomes, obtainable in that
(a) actives are brought into contact with agents capable to form liposomes, and
(b) the liposomes thus obtained are treated with polymers or surfactants showing the opposite charge of said liposomes to form capsules.

Surprisingly it has been observed that the capsules according to the present invention serve the needs of the market in a perfect way. First, the capsules show an average diameter of about 10 to about 900 nm, and preferably of about 200 to about 400 nm. As one can see from spectroscopic measurements, the products exhibit a narrow particle size distribution indicating that at least 60, but usually at least 70 % of the particles show the preferred size. The shell around the actives is formed by coazervation of a liposome and a polymer showing the opposite charge. Usually a liposome with a negative charge is combined with a cationic polymer or cationic surfactant. Determination of the zeta potential show that under these circumstances the capsules are negatively charged what makes it easier to bind them to fibres, either keratin fibres of hair or synthetic fibres of textiles. Since the liposomes represent a lipophilic phase another condition is fulfilled: when the capsule breaks the active is released however still embedded in the lipophilic liposmal phase, so that also active showing only little hydrophobicity can be transported through the skin barrier. Finally, the capsules are found to be rather stable even in the presence of anionic surfactants, however break easily when subjected to mechanic pressure, for example when a cream or shampoo comprising said capsules are administered to skin or hair.

### Manufacturing

Another object of the present invention refers to a method for producing encapsulated liposomes, which are obtained by
(a) bringing actives into contact with agents capable to form liposomes, and
(b) treating the liposomes thus obtained with polymers or surfactants showing the opposite charge of said liposomes to form capsules.

More precisely this means that in case one uses negatively charged liposomes than positively charged polymers or surfactants are necessary and vice-versa.

### Actives

The selection of actives is not critical for the present invention, since in principle the disclosed process can be applied to each type of active, although lipophilic actives are preferred. Typical examples - not limiting the present invention - include oil bodies, primary and secondary sun protection factors, biogenic agents, perfume oils and dyes.
o Suitable **oil bodies,** which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C ₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.
o **Primary sun protection factors** in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
   - 3-benzylidene camphor or 3-benzylidene norcamphor and derivatives thereof, for example 3-(4-methylbenzylidene)-camphor;
   - 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
   - esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
   - esters of salicylic acid, preferably salicylic acid-2-ethylhexyl ester, salicylic acid-4-isopropylbenzyl ester, salicylic acid homomenthyl ester;
   - derivatives of benzophenone, preferably 2-hydroxy-4-methoxybenzophenone, 2-hydroxy-4-methoxy-4'-methylbenzophenone, 2,2'-dihydroxy-4-methoxybenzophenone;
   - esters of benzalmalonic acid, preferably 4-methoxybenzalmalonic acid di-2-ethylhexyl ester;
   - triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb^{®} HEB);
   - propane-1,3-diones such as, for example, 1-(4-tert.butylphenyl)-3-(4'-methoxyphenyl)-propane-1,3-dione;
   - ketotricyclo(5.2.1.0)decane derivatives.
   Suitable water-soluble substances are
   • 2-phenylbenzimidazole-5-sulfonic acid and alkali metal, alkaline earth metal, ammonium, alkylammonium, alkanolammonium and glucammonium salts thereof;
   • sulfonic acid derivatives of benzophenones, preferably 2-hydroxy-4-methoxybenzophenone-5-sulfonic acid and salts thereof;
   • sulfonic acid derivatives of 3-benzylidene camphor such as, for example, 4-(2-oxo-3-bomylidenemethyl)-benzene sulfonic acid and 2-methyl-5-(2-oxo-3-bornylidene)-sulfonic acid and salts thereof.
   Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.
o Besides the groups of primary sun protection factors mentioned above, **secondary sun protection factors** of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-camosine, D-camosine, L-camosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta- , hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).
o In the context of the invention, **biogenic agents** are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils for example moringa oil, plant extracts, for example prune extract, bambara nut extract, and vitamin complexes.
o Suitable **perfume oils** are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, 0-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihy-dromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.
o Suitable **dyes** are any of the substances suitable and approved for cosmetic purposes as listed, for example, in the publication **"Kosmetische Färbemittel"** of the Farbstoff-kommission der Deutschen Forschungsgemeinschaft, **Verlag Chemie, Weinheim, 1984, pages 81 to 106**. Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1 % by weight, based on the mixture as a whole.

### Liposome forming agents

Among the group agents capable to form liposomes lecithins and phospholipids are the most preferred ones, due to their price and outstanding properties. Usually the actives are dissolved in a suitable solvent and than brought into contact with said liposome forming agents at temperatures within a range of 30 to 70 and preferably about 50 °C. It is however also possible to add the non-aqueous actives to the solutions of the lecithins or phospholipids. Typical examples are phosphatidyl choline, phosphatidyl glycerol and cholesterol. Typically, actives and liposome forming agents are used in a ratio by weight of about 1:20 to about 5:1 and preferably about 1:2 to 4:1. Suitable solvents are lower alcohols such as ethanol, and polyols having 2 to 15 carbon atoms and at least two hydroxyl groups. The most preferred solvent is propylene glycol.

In a preferred embodiment of the present invention lecithins or phospholipids are used to form negatively charged liposomes and cationic polymers or cationic surfactants to form the capsules.

### Cationic polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Preferred cationic polymers are cationic chitin derivatives such as, for example chitosan, optionally in microcrystalline distribution. Chitosans are biopolymers which belong to the group of hydrocolloids. Chemically, they are partly de-acetylated chitins differing in their molecular weights which contain the following - idealized - monomer unit:

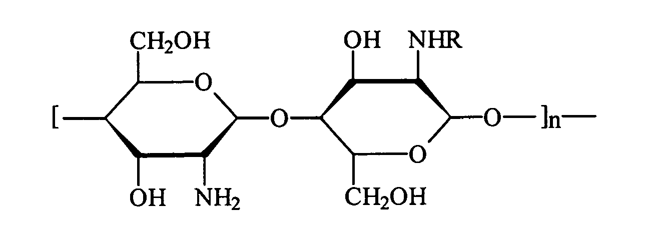

In contrast to most hydrocolloids, which are negatively charged at biological pH values, chitosans are cationic biopolymers under these conditions. The positively charged chitosans are capable of interacting with oppositely charged surfaces and are therefore used in cosmetic hair- care and body-care products and pharmaceutical preparations. Chitosans are produced from chitin, preferably from the shell residues of crustaceans which are available in large quantities as inexpensive raw materials. In a process described for the first time by Hackmann et al., the chitin is normally first de-proteinized by addition of bases, de-mineralized by addition of mineral acids and, finally, de-acetylated by addition of strong bases, the molecular weights being distributed over a broad spectrum. Preferred types are those which are disclosed in German patent applications DE 4442987 A1 and DE 19537001 A1 (Henkel) and which have an average molecular weight of 10,000 to 500,000 Dalton or 800,000 to 1,200,000 Dalton and/or a Brookfield viscosity (1 % by weight in glycolic acid) below 5,000 mPas, a degree of de-acetylation of 80 to 88 % and an ash content of less than 0.3% by weight. In the interests of better solubility in water, the chitosans are generally used in the form of their salts, preferably as glycolates.

### Cationic surfactants

Also monomeric cationic surfactants are capable to interact with negative charged liposomes to form a capsule. The preferred types are esterquats and tetraalkylammonium salts. The most preferred species, however, belong to the so-called "polymeric esterquats", surfactants combining surfactant and polymer performance in one molecule. Polymeric esterquats are obtained by reacting alkanol amines with (mono) fatty acids and dicarboxylic acids, and quaternising the resulting esters with alkylation agents in known manner, optionally after alkoxylation.

According to the present invention, suitable polymeric esterquats are derived from alkanolamines derived from amines following general formula (I). 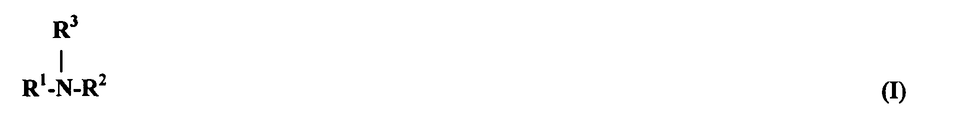 in which R¹ represents a hydroxyethyl radical, and R² and R³ independently of one another stand for hydrogen, methyl or a hydroxyethyl radical. Typical examples are methyldiethanolamin (MDA), monoethanolamine (MES), diethanolamine (DEA) and triethanolamine (TEA). In a preferred embodiment of the present invention, triethanolamine is used as the starting material.

On the other hand, in a further preferred embodiment of the present invention, it is possible to use mixtures of
(i) Monocarboxylic acids selected from the group consisting of caproic acid, caprylic acid, 2-ethyl hexanoic acid, caprinic acid and their mixtures,
(ii) Monocarboxylic acids selected from the group consisting of lauric acid, myristic acid, palmitic acid, stearic acid, oleic acid, behenic acid, erucic acid and their mixtures, and
(iii) Dicarboxylic acids according to general formula **(II)**,

   **HOOC-[X]-COOH** **(II)**

   in which [X] stands for an optionally hydroxysubstituted alk(en)ylene group having 1 to 10 carbon atoms.

It has to be understood that the fatty acids representing group (i) and (ii) may also encompass technical grade fatty acids mixtures which can be derived from the splitting of fats and oils, optionally after additional separation and distillation, and therefore may also include other species.

Dicarboxylic acids (iii) suitable for use as starting materials in accordance with the invention are typically selected from the group consisting of succinic acid, maleic acid, glutaric acid, 1,12-dodecanedioic acid. The best results, however, are obtained by incorporating adipic acid into the polymeric esterquat. The over-all preferred polymeric esterquats are obtained from mixtures consisting of caprylic acid, stearic acid and adipic acid.

With respect to the properties, specially related to elasticity and stability of the capsules in the final products it has been found rather advantageous to use the monocarboxylic acids forming the groups (i) and (ii) in molar ratios of about 30:70 to about 70:30, and preferably in a ratio of about 50:50.

The fatty acids (i+ii) and the dicarboxylic acids (iii) may be used in a molar ratio of 1:10 to 10:1. However, it has proved to be of advantage to adjust a molar ratio of 1:1 to 2:1. The trialkanolamines on the one hand and the acids - i.e. fatty acids and dicarboxylic acids together - on the other hand may be used in a molar ratio of 1:1 to 1:2. A molar ratio of trialkanolamine to acids of 1:1.2 to 1:1.5 has proved to be optimal. The esterification may be carried out in known manner, for example as described in International patent application WO 91/01295 (Henkel). In one advantageous embodiment, it is carried out at temperatures between 120 °C and 220 °C, and more particularly between 130 °C and 170 °C under pressures of 0.01 to 1 bar. Suitable catalysts are hypophosphorous acids and alkali metal salts thereof, preferably sodium hypophosphite, which may be used in quantities of 0.01 to 0.1% by weight, and preferably in quantities of about 0.05 to about 0.07 % b.w. based on the starting materials. In the interests of particularly high colour quality and stability, it has proved to be of advantage to use as co-catalysts alkali metal and/or alkaline earth metal borohydrides, for example, potassium, magnesium and, in particular, sodium borohydride. The co-catalysts are normally used in quantities of about 50 to about 1.000 ppm, and more particularly in quantities of about 100 to about 500 ppm, again based on the starting materials. Corresponding processes are also the subject of DE 4308792 C1 and DE 4409322 C1 (Henkel) to which reference is hereby specifically made. Alternatively, the esterification may be carried out with the two components in successive steps.

The quaternisation of the fatty acid/dicarboxylic acid trialkanolamine esters may be carried out in known manner. Although the reaction with the alkylation agents may also be carried out in the absence of solvents, it is advisable to use at least small quantities of water or lower alcohols, preferably isopropyl alcohol, for the production of concentrates which have a solids content of at least 80% by weight, and more particularly at least 90% by weight. Suitable alkylation agents are alkyl or aryl halides such as, for example, methyl chloride, benzyl chloride dialkyl sulphates, such as dimethyl sulphate or diethyl sulphate, for example, or dialkyl carbonates, such as dimethyl carbonate or diethyl carbonate, for example. The esters and the alkylating agents are normally used in amounts of 95 to 105 Mol-% calculated on the molar amount of nitrogen within the ester mixture, i.e. in a substantially stoichiometric ratio. The reaction temperature is usually in the range from 40 °C to 80 °C, and more particularly in the range from 50 °C to 60 °C. After the reaction it is advisable to destroy unreacted alkylation agent by addition of, for example, ammonia, an (alkanol)amine, an amino acid or an oligopeptide as described, for example, in DE 14026184 A1 (Henkel).

### Formation of liposomes and encapsulation

The formation of the liposomes has already been described above. After its preparation from actives and liposome forming agents, the liposomes are very finely dispersed optionally in an oil phase with intensive shearing in order to produce small particles in the subsequent encapsulation process. It has proved to be particularly advantageous in this regard to heat the liposomes to temperatures in the range from 40 to 60° C while the oil phase is cooled to 10 to 20° C. The actual encapsulation, i.e. formation of the membrane by contacting the cationic polymer in the liposomes, takes place in a second step. To this end, it is advisable to wash the liposomes - optionally dispersed in the oil phase - with an aqueous ca. 0.1 to 3 and preferably 0.25 to 0.5% by weight solution of the cationic polymer or cationic surfactant, at a temperature in the range from 40 to 100 and preferably 50 to 60° C. and, at the same time, to remove the oil phase if present. In the alternative the liposomes can be dropped into a solution of said polymers or surfactants. The resulting - aqueous - preparations generally have a microcapsule content of 1 to 10% by weight. In some cases, it can be of advantage for the solution of the polymers to contain other ingredients, for example emulsifiers or preservatives. After filtration, the encapsulated liposomes are obtained. It is advisable to sieve the capsules to ensure a uniform size distribution. The microcapsules thus obtained may have any shape within production-related limits, but are preferably substantially spherical.

### Industrial application

Due to their outstanding performance the encapsulated liposomes are useful for a broad range of applications. Therefore, further embodiments of the present invention are related to the use of the capsules for making
o Cosmetic and/or pharmaceutical compositions, preferably skin care hair care or personal care compositions.
o Detergent compositions, preferably manual dish wash compositions and light duty detergents
o Food compositions., preferably functional food or dietary supplements to be incorporated for example in beverages or milk products;
o Textile or paper additives.
o Lacquers and paints.

### Examples

### Example 1

2 g of soy lecithin and 10 g propylene glycol were placed in a 100 ml flask, filled up with water to a volume of 70 ml and heated to about 70°C. Under vigorous stirring 5 g of hydrolyzed ceratine (Cashmilan^{®} LS 9960, Cognis France) were added until a homogenous mixture was achieved. Finally, the product was treated with 3 g PEG-15 Cocopropylamine in 11 g water and 1 g of preservative (Phenonip^{®}). The resulting product comprised nanocapsules having an average diameter of 200 to 300 nm (measured by Photon Correlation Spectroscopy).

In the following Figure 1 Zeta potential versus Intensity is shown. The peak in the middle represents the average Zeta potential of 15 mV, which indicates that the capsules are positively charged.

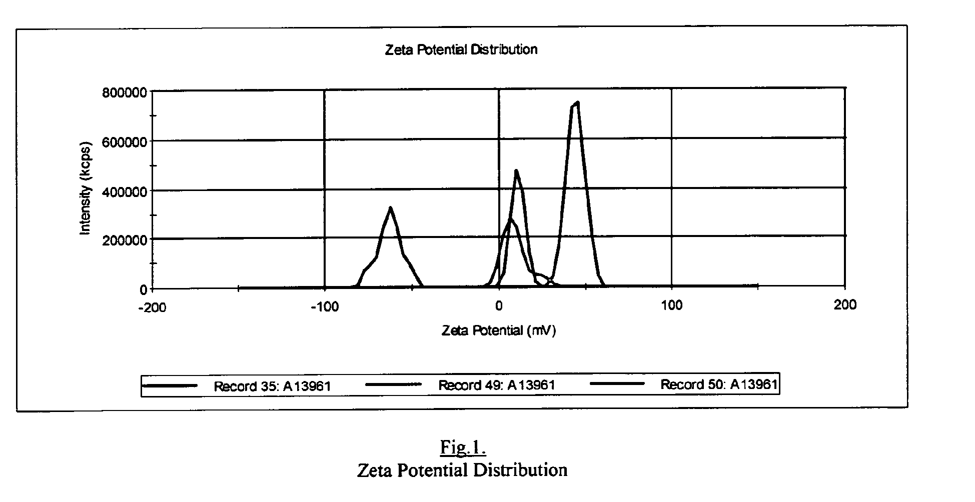

### Example 2

2.25 g phoisphatidyl choline, 0.25 g cholesterol and 12 g propylene glycol were placed in a 100 ml flask, filled up with water to a volume of 80 ml and heated to about 75°C. Under vigorous stirring 5 g of retinol were added until a homogenous mixture was achieved. Finally, the product was treated with 3 g PEG-15 Cocopropylamine in 11 g water and 1 g of preservative (Phenonip^{®}). The resulting product comprised nanocapsules having an average diameter of 250 to 300 nm (measured by Photon Correlation Spectroscopy).

### Example 3

2 g of soy lecithin and 10 g propylene glycol were placed in a 100 ml flask, filled up with water to a volume of 70 ml and heated to about 70°C. Under vigorous stirring 5 g of Moringa oil (Lipofructyl^{®}, Cognis France) were added until a homogenous mixture was achieved. Finally, the product was treated with 3 g of a polymeric esterquat with asymmetric side chains in 11 g water and 1 g of preservative (Phenonip^{®}). The resulting product comprised nanocapsules having an average diameter of 180 to 250 nm (measured by Photon Correlation Spectroscopy).

## Claims

1. Encapsulated liposomes, obtainable in that
(a) actives are brought into contact with agents capable to form liposomes, and
(b) the liposomes thus obtained are treated with polymers or surfactants showing the opposite charge of said liposomes to form capsules.

2. Encapsulated liposomes according to Claim 1, **characterised in that** said capsules show an average diameter of 10 to 900 nm.

3. Encapsulated liposomes according to Claims 1 and/or 2, **characterised in that** said capsules show an average diameter of 100 to 1.200 nm.

4. A method for producing encapsulated liposomes, **characterised in that**
(a) actives are brought into contact with agents capable to form liposomes, and
(b) the liposomes thus obtained are treated with polymers or surfactants showing the opposite charge of said liposomes to form capsules.

5. Method according to Claim 4, **characterised in that** one uses negatively charged liposomes and positively charged polymers or surfactants, or vice-versa.

6. Method according to any of the Claims 4 to 5, **characterised in that** said actives are selected from the group consisting of oil bodies, primary and secondary sun protection factors, biogenic agents, perfume oils and dyes.

7. Method according to any of the Claims 4 to 6, **characterised in that** said agents capable to form liposomes are selected from the group consisting of lecithins and phospholipids.

8. Method according to any of the Claims 4 to 7, **characterised in that** said agents capable to form liposomes are dissolved in ethanol or polyols.

9. Method according to any of the Claims 4 to 8, **characterised in that** one uses lecithins or phospholipids to form negatively charged liposomes and cationic polymers or cationic surfactants to form the capsules.

10. Method according to any of the Claims 4 to 9, **characterised in that** said cationic polymers are selected from the group consisting of quaternized hydroxyethyl cellulose, cationic starch, copolymers of diallyl ammonium salts and acrylamides, quaternized vinyl pyrrolidone/vinyl imidazole polymers, condensation products of polyglycols and amines, quaternized collagen polypeptides, quaternized wheat polypeptides, polyethyleneimine, cationic silicone polymers, copolymers of adipic acid and dimethylaminohy-droxypropyl diethylenetriamine, copolymers of acrylic acid with dimethyl diallyl ammonium chloride, polyaminopolyamides and crosslinked water-soluble polymers thereof, condensation products of dihaloalkyls, cationic guar gum, quaternized ammonium salt polymers, polyquaternium types and chitosan.

11. Method according to any of the Claims 4 to 10, **characterised in that** said cationic surfactants are selected from the group consisting of esterquats and tetraalkylammonium salts.

12. Use of encapsulated liposomes according to Claim 1 for making cosmetic and/or pharmaceutical compositions.

13. Use according to Claim 12, **characterised in that** said compositions represent skin care hair care or personal care compositions.

14. Use of encapsulated liposomes according to Claim 1 for making detergent compositions.

15. Use of encapsulated liposomes according to Claim 1 for making food compositions.

16. Use of encapsulated liposomes according to Claim 1 for making textile or paper additives.

17. Use of encapsulated liposomes according to Claim 1 for making lacquers and paints.
